Europäisches Patentamt

(19) European Patent Office

Office européen des brevets

(11) Publication number: **0 293 127 B1**

(12) # EUROPEAN PATENT SPECIFICATION

(45) Date of publication of patent specification:
23.10.91 Bulletin 91/43

(51) Int. Cl.⁵: **C07C 59/153, C07C 51/34**

(21) Application number: **88304466.1**

(22) Date of filing: **17.05.88**

(54) Process for preparing glyoxylic acid.

(30) Priority: **27.05.87 JP 128445/87**

(43) Date of publication of application:
**30.11.88 Bulletin 88/48**

(45) Publication of the grant of the patent:
**23.10.91 Bulletin 91/43**

(84) Designated Contracting States:
**AT DE FR GB**

(56) References cited:
**EP-A- 0 099 981**
**GB-A- 1 378 351**
**US-A- 3 145 232**

(73) Proprietor: **MITSUI TOATSU CHEMICALS INC.**
**No. 2-5, Kasumigaseki 3-chome**
**Chiyoda-Ku Tokyo 100 (JP)**

(72) Inventor: **Arashiba, Nobumasa**
**No. 2-246, Shinke 3-9, Nishitoriishi**
**Takaishi-shi Osaka-fu (JP)**
Inventor: **Asano, Shiro**
**No. 450-15, Kuden-cho Sakae-ku**
**Yokohama-shi Kanagawa-ken (JP)**
Inventor: **Ono, Yasuko**
**No. 3-343, Ayai 4-7, Kamo**
**Takaishi-shi Osaka-fu (JP)**

(74) Representative: **Hayward, Denis Edward Peter et al**
**Lloyd Wise, Tregear & Co. Norman House**
**105-109 Strand**
**London WC2R 0AE (GB)**

EP 0 293 127 B1

## Description

The present invention relates to a process for preparing glyoxylic acid by ozonolysis of maleic acid derivatives.

Glyoxylic acid contains an aldehyde group and a carboxyl group in its molecule, and is highly reactive. Therefore glyoxylic acid and its derivatives are very important compounds as intermediates for the preparation of various chemicals such as drug modifiers, cosmetics, perfumes and agricultural chemicals.

Various processes have been known as the preparation process of glyoxylic acid. The processes include, recovery as a by-product of glyoxal in the nitric acid oxidation process of acetaldehyde, oxidizing glyoxal with nitric acid, chlorine or by electro-chemistry, the electrochemical reduction of oxalic acid and the ozonation of maleic acid.

As to the process for preparing glyoxylic acid by the ozonolysis of maleic acid, a method for ozonizing maleic acid in water is described in Ind. Eng. Chem., Prod. Res. Develop., 5, (4), 350-351 (1966). In this process, one mole each of glyoxylic acid, formic acid and carbon dioxide are generated from one mole of maleic acid. Even if the reaction proceeds ideally, the yield of glyoxylic acid is 50% based on carbon atoms of maleic acid. U.S. Pat. 3,637,721 discloses a process for obtaining carbonyl compounds by ozonizing the carbon-carbon double bond, followed by reducing with a sulfide compound such as dimethyl sulfide at a temperature of from −70 to 50°C. In this process, the ozonolysis of maleic acid to formic acid and carbon dioxide does not occur, and theoretically two moles of glyoxylic acid are formed from one mole of maleic acid. Thus the desired product can be obtained in a yield of 90% and more. The sulfide compound, however, is very expensive as a reducing agent and the sulfoxide formed by the reduction is difficult to separate from the desired product. U.S. Pat. 3,705,922 teaches a process for ozonizing maleic acid in methanol at a temperature of −10°C or less, followed by hydrogenating the ozonolysis products in the presence of an alumina supported palladium catalyst to obtain glyoxylic acid methyl hemiacetal. The hydrogenation process is advantageous in that the reducing agent is inexpensive and furthermore glyoxylic acid methyl hemiacetal can be obtained in a good yield.

The process of ozonizing maleic acid and subsequently reducing the ozonolysis products is an excellent process as mentioned above. In this process, however, the carriers of the palladium catalyst used for the hydrogenation are limited as described in U.S. Pat. 3,705,922. The process also has the problem that the optimum ranges for the supported amount of palladium and the amount of the catalyst used are very narrow and the reaction is unstable. Therefore, it is desirable to improve the hydrogenation process for obtaining glyoxylic acid. In addition, a decrease in the yield of glyoxylic acid which is the desired product of the reaction has resulted from the formation of formic acid and oxalic acid in considerable quantities as by-products. It is desirable to inhibit the formation of these by-products and to enhance the selectivity for glyoxylic acid.

The present inventors have intensively investigated the process for obtaining glyoxylic acid by the ozonolysis of maleic acid in order to overcome the problems mentioned above. As a result, it has been found that when hydrogenation is conducted in the presence of a generally used hydrogenation catalyst, the desired product can be obtained in a good yield if an antioxidant is present.

Accordingly, the present invention provides a process for preparing glyoxylic acid comprising :

a) dissolving a maleic acid derivative selected from maleic acid, maleic anhydride, monoalkyl maleate and dialkyl maleate in a lower alkanol ;

b) reacting said dissolved maleic acid derivative with an excess of ozone at a temperature not above 10°C to give an oxidized compound ;

c) hydrogenating said oxidized compound in the presence of an antioxidant at a temperature not above 15°C over a transition metal catalyst or a platinum group metal catalyst to give glyoxylic acid hemiacetal ;

d) filtering the reaction mixture to remove the catalyst ;

e) reacting the resultant hemiacetal with an excess of water after or without removing the solvent ; and

f) evaporating the excess water or the excess water and solvent.

In this process, glyoxylic acid hemiacetal includes such obtained in the form of glyoxylic acid esters.

The practice of the present invention will hereinafter be described in more detail.

The maleic acid derivative employed in the process of this invention may be an industrially produced and commercially available grade of maleic acid prepared by the hydrolysis of maleic anhydride which is industrially easy to obtain.

The lower alkanol solvent used may be selected from alcohols such as methyl alcohol, ethyl alcohol, n-propyl alcohol, isopropyl alcohol, n-butyl alcohol, sec-butyl alcohol, and iso-butyl alcohol. These alcohols may be used alone or as a mixture of two or more. When maleic anhydride is dissolved in the alcoholic solvent, maleic acid monoalkyl ester having a corresponding alkyl group is formed. The monoalkyl ester can be converted to glyoxylic acid by ozonation, hydrogenation and subsequent hydrolysis. the maleic acid can be reacted in a concentration of about 0.1–40 weight percent. A concentration less than 0.1 weight percent gives poor pro-

2

duction efficiency and is unfavorable from the practical standpoint. The reaction carried out at a concentration of more than 40 weight percent is also unfavorable because of reduced selectivity in the reaction.

Ozone used for ozonolysis can be generated by an ozonizer and its oxygen source may be air or oxygen. The ozone used may be in the same molar amount as the maleic acid and 1.1-1.5 times by mole of ozone is the most preferred because some quantity of unreacted ozone escapes from the reaction system. Almost 100% of the maleic acid can be converted by the ozonolysis. The reaction temperature is 10°C or less, and a more preferred temperature is 0°C or less. However, it is not necessary to lower the temperature as far as below −50°C which is a temperature that can be attained by using an ordinary freezing mixture such as solid carbon dioxide/acetone or solid carbon dioxide/methanol. The preferred range is from −5°C to −50°C. Hydrogenation is carried out after completing the ozonolysis.

The hydrogenation catalyst used in the process of this invention is conventional and may be a transition metal such as nickel, iron, cobalt or copper in the form of powder or Raney metal, or a platinum group metal catalyst such as palladium, platinum, rhodium, ruthenium, or iridium. These metals may be supported on carriers such as alumina, silica, silica-alumina and activated carbon. The amount of the catalyst used is varied depending upon the kind of metal, the quantity supported on the carrier and the hydrogen pressure. The amount is usually 0.1-10 weight percent of the reaction mass.

Any industrially available hydrogen may be used for the hydrogenation. When the hydrogenation reaction is carried out in the presence of an antioxidant as in the process of this invention, high selectivity can be achieved under both elevated and ambient pressures of hydrogen.

The hydrogen temperature is 15°C or less. A more preferred temperature is in the range of 5°C to −40°C. Temperatures above 15°C are unfavourable because the intermediate formed by the ozonolysis of maleic acid decomposes spontaneously and greatly reduces the selectivity of hydrogenation. Temperatures below −40°C are not preferred because the reaction rate becomes extremely slow and completion of the reaction also becomes difficult. The reaction time required for hydrogenation is 0.5-10 hours and varies depending upon the kind of catalyst used and the concentration of maleic acid derivatives in the solvent.

The antioxidant which should be present during the hydrogenation process of this invention is selected from organic compounds providing various autoxidative materials with the property of preventing or inhibiting the action of oxygen under light radiation or heat. The antioxidants generally include all compounds which are roughly classified from their action mechanism into free radical chain terminators, peroxide decomposers and metal deactivators. Free radical chain terminators and peroxide decomposers prevent oxidation by directly participating in an oxidising reaction. They are antioxidants in a narrow sense. Typical examples are quinones, aromatic amines and phenols. Suitable compounds which may be used include, for example, quinones such as hydroquinone, hydroquinone monomethyl ether and naphthoquinone ; amines such as phenothiazine, alpha-naphthylamine, N-phenyl-beta-naphthylamine, N,N′-diphenyl-p-phenylenediamine and N,N′-di-sec-butyl-p-phenylenediamine ; and phenols such as 2,6-di-tert-butyl-p-cresol, 2,6-di-tert-butylphenol, 2,4-dimethyl-6-tert-butylphenol, butylhydroxyanisole and 2,2′-methylenebis(4-methyl-6-tert-butylphenol). The metal deactivators are sulfur compounds, phosphorous compounds and sulfur-phosphorous compounds. Representative deactivators which may be used include, for example, sulfur compounds such as thioalkylphenol, its calcium salt and thioterpene ; phosphorous compounds such as tributyl phosphite, and triphenyl phosphite; and sulfurphosphorous compounds such as calcium dithiophosphate. Some of these metal deactivators, however, deactivate the catalytic action of platinum group metals and transition metals which are used as the catalyst. Sometimes these deactivators are also a little less effective as compared with the above mentioned free radical chain terminators or peroxide decomposers.

The preferred amount of the antioxidant used is in the range of 0.1 part per million to 1 weight percent based on the amount of maleic acid derivatives. An amount less than 0.1 part per million gives little effect. When the antioxidant is used in a concentration above 1 weight percent, there is no adverse effect on the reaction but no advantage is found in particular. Furthermore, many of the antioxidants are expensive and it is wasteful to use an amount more than is needed.

A marked advantage obtained by conducting the hydrogenation reaction in the presence of the antioxidant is that formation of oxalic acid by-product is remarkably inhibited. The yield of glyoxylic acid can be markedly improved as a result of inhibiting oxalic acid formation.

In the process of this invention, the antioxidant should be present at least during the hydrogenation period and may be added prior to the ozonisation reaction or after the termination thereof. In some conditions, the antioxidant itself is decomposed by ozone or the hydrogenation catalyst is poisoned by a substance generated by the decomposition of antioxidant. Therefore the antioxidant is preferably added after completion of ozonolysis.

The product can be obtained as an alcoholic solution by filtering off the catalyst after completing the hydrogenation reaction. In this case, the desired product, the glyoxylic acid derivative exists as hemiacetal. Thus the hemiacetal of the glyoxylic acid derivative can be obtained by removing the solvent. Glyoxylic acid can be obtained in the form of monohydrate by hydrolyzing the glyoxylic acid hemiacetal with an excess amount of water after or without removing alcohol, followed by removing water or water and alcohol.

EXAMPLES

The practice of the present invention will hereinafter be further illustrated by detailed examples, which are not, however, limiting upon the scope of the invention.

Example 1

A solution of 10.8 g (93 m.mole) of maleic acid in 100 ml of methanol was charged into a glass tube having an internal diameter of 27 mm and equipped with a glass filter at the bottom. The internal temperature was maintained at –45°C to –40°C with a solid carbon dioxide/acetone bath. Oxygen gas containing about 0.93 vol% of ozone was introduced into the solution through the glass filter at a rate of 2 1/min for 2 hours. The introduced amount of ozone was 1.1 moles per mole of maleic acid. After completing ozonolysis, nitrogen gas was introduced into the reaction mixture at a rate of 1 1/min for 15 minutes at the same internal temperature as above. Then a solution of 5.4 mg (500 ppm) of phenothiazine in about 5 ml of methanol was added to the reaction mixture, followed by adding 0.6 g of a catalyst powder consisting of 5 wt% of palladium supported on alumina. The internal temperature was raised to 10°C during about 1.5 hours while introducing hydrogen gas at a rate of 0.7 1/min. Hydrogen gas was further introduced for an hour at the same temperature. After completing hydrogenation, nitrogen gas was introduced for 2 minutes and the catalyst was filtered off.

The resulting reaction mixture had added to it about 300 ml of water and methanol was removed at 50°C under reduced pressure in a rotary evaporator. At a suitable time in the course of the evaporation process, 300 ml of water was added twice to carry out hydrolysis sufficiently. The aqueous solution of glyoxylic acid thus obtained was about 300 ml. After precisely weighing out 1 g of the solution, it was analysed by high speed liquid chromatography in accordance with the interoffice standard method. The separation column was SHODEX KC-811 and the moving phase was an aqueous 0.05 vol.% phosphoric acid. The analytical results obtained were a maleic acid conversion of 100%, glyoxylic acid selectivity of 92% (171 m.mole) and oxalic acid selectivity of 2.2% (3.8 m.mole). A peroxide test with a potassium iodide/starch paper was negative immediately after terminating hydrogenation and it was found that hydrogenation was completely carried out.

Comparative Example 1

Ozonolysis of maleic acid in methanol and introduction of nitrogen gas into the reaction mixture were conducted by the same procedures as Example 1. Then the addition of phenothiazine was omitted and 0.6 g of the same catalyst powder, 5 wt.% of palladium supported on alumina, as in Example 1 was added alone. The internal temperature was raised to 10°C during 1.5 hours while introducing hydrogen gas at a rate of 0.7 1/min. Hydrogen gas was further introduced for an hour at the same temperature. After completing hydrogenation, the catalyst was filtered off. The resulting reaction mixture was hydrolyzed by the same procedures as in Example 1 and subjected to analysis. The analytical results obtained were a maleic acid conversion of 100%, glyoxylic acid selectivity of 80% (149 m.mole) and oxalic acid selectivity of 8.1% (14.9 m.mole). A peroxide test with a potassium iodide/starch paper was negative immediately after terminating hydrogenation.

Example 2

The same procedures as in Example 1 were carried out except that 0.6 g of a catalyst powder consisting of 5 wt.% of palladium supported on activated carbon was used as the hydrogenation catalyst and 10.8 mg (1,000 ppm) of phenothiazine was used as the antioxidant. Post hydrogenation was also conducted by the same procedures as in Example 1 and the product was analysed. The analytical results obtained were a maleic acid conversion of 100%, glyoxylic acid selectivity of 93% (173 m.mole) and oxalic acid selectivity of 2.0% (3.7 m..mole). The peroxide test was negative.

Comparative Example 2

The same procedures as in Example 2 were carried out without using phenothiazine as an antioxidant. The analytical results of the resulting product were a maleic acid conversion of 100%, glyoxylic acid selectivity of 82% (150 m.mole) and oxalic acid selectivity of 8.6% (15.7 m.mole). A peroxide test was negative at the termination of hydrogenation.

## Example 3

The same procedures as in Example 1 were carried out except that 5.4 mg (500 ppm) of phenyl-beta-naphthylamine was used as the antioxidant. The post hydrogenation treatment of Example 1 was repeated. The analytical results of the product obtained were a maleic acid conversion of 100%, glyoxylic acid selectivity of 90% (168 m.mole) and oxalic acid selectivity of 2.5% (4.7 m.mole). The peroxide test was negative.

## Example 4

The same procedures as in Example 1 were carried out except that 1.08 mg (1,000 ppm) of alpha-naphthylamine was used as the antioxidant. The analytical results of the product obtained were a maleic acid conversion of 100%, glyoxylic acid selectivity of 92% (171 m.mole) and oxalic acid selectivity of 3.0% (5.5 m.mole). The peroxide test was negative.

## Example 5

The procedures of Example 1 were repeated except that 0.6 g of a catalyst powder consisting of 5 wt.% of platinum supported on activated carbon was used as the hydrogenation catalyst and 10.8 mg (1,000 ppm) of 2,6-di-tert-butyl-p-cresol was used as the antioxidant in place of phenothiazine. The same post hydrogenation treatment was carried out as in Example 1. The analytical results of the product obtained were a maleic acid conversion of 100%, glyoxylic acid selectivity of 59% (108 m.mole) and oxalic acid selectivity of 1.9% (3.5 m.mole). The peroxide test was negative.

## Comparative Example 3

The same procedures as in Example 5 were carried out without using 2,6-di-tert-butyl-p-cresol as an antioxidant.
The analytical results of the product obtained were a maleic acid conversion of 100%, glyoxylic acid selectivity of 48% (89 m.mole) and oxalic acid selectivity of 7.9% (14.6 m.mole). A peroxide test was negative at the termination of hydrogenation.

## Example 6

The procedures of Example 1 were repeated except that 0.5 g of Raney nickel catalyst dipped in methanol was used as the hydrogenation catalyst in place of the palladium catalyst and 54 mg (500 ppm) of 2,4-dimethyl-6-tert-butylphenol was used as the antioxidant in place of phenothiazine. The same post hydrogenation treatment was conducted as in Example 1. The analytical results of the product obtained were a maleic acid conversion of 100%, glyoxylic acid selectivity of 62% (113 m.mole) and oxalic acid selectivity of 3.8% (6.9 m.mole). The peroxide test was negative.

## Comparative Example 4

The same procedures as in Example 6 were carried out without using 2,4-dimethyl-6-tert-butylphenol as an antioxidant. The analytical results of the product obtained were a maleic acid conversion of 100%, glyoxylic acid selectivity of 53% (97 m.mole) and oxalic acid selectivity of 6.8% (12 m.mole). The peroxide test was negative.

## Example 7

The same procedures as in Example 1 were carried out except that 5.4 mg (500 ppm) of hydroquinone was used as the antioxidant. The same post hydrogenation treatment was conducted as in Example 1. The analytical results of the product obtained were a maleic acid conversion of 100%, glyoxylic acid selectivity of 84% (154 m.mole) and oxalic acid selectivity of 4.3% (7.9 m.mole). The peroxide test was negative.

### Example 8

The same procedures as in Example 1 were carried out except that 5.4 mg (500 ppm) of thioalkylphenol was used as the antioxidant. The results were a maleic acid conversion of 100%, glyoxylic acid selectivity of 80% (146 m.mole) and oxalic acid selectivity of 6.7% (12.3 m.mole). A peroxide test was negative at the termination of hydrogenation.

### Example 9

The same procedures as in Example 1 were carried out except that 1.08 mg (100 ppm) of triphenyl phosphite was used as the antioxidant. The results were a maleic acid conversion of 100%, glyoxylic acid selectivity of 82% (150 m.mole) and oxalic acid selectivity of 6.3% (11.5 m.mole). The peroxide test was negative.

### Example 10

The procedures of Example 2 were repeated except that the ozone was introduced at a temperature of −20°C. The results were a maleic acid conversion of 100%, glyoxylic acid selectivity of 91% (169 m.mole) and oxalic acid selectivity of 2.3%. A peroxide test was negative at the termination of hydrogenation.

### Example 11

The procedures of Example 1 were repeated except that the ozone was introduced at a temperature of −10°C and 1.08 mg (100 ppm) of alpha-naphthylamine was used as the antioxidant. The results were a maleic acid conversion of 100%, glyoxylic acid selectivity of 90% and oxalic acid selectivity of 3.5%. The peroxide test was negative.

The results of the above Examples and Comparative Examples are summarised in Table 1.

As described above in detail, the process of this invention is based on finding out the fact that the formation of oxalic acid by-product can be markedly inhibited if after ozonizing maleic acid derivatives in alcohol the ozonized intermediates are hydrogenated using an ordinary hydrogenation catalyst in the presence of an antioxidant. As a result, glyoxylic acid hemiacetal can be obtained in extremely high yield and the reaction is very stable.

Accordingly, the present invention is very easy to carry out industrially and can provide glyoxylic acid and its hemiacetal in a high yield.

Table 1

| Example | Antioxidant | Amount (ppm) | Catalyst | Maleic acid conversion (%) | Selectivity (%) | |
|---|---|---|---|---|---|---|
| | | | | | Glyoxylic acid | Oxalic acid |
| Example 1 | Phenothiazine | 500 | 5% Pd/Al$_2$O$_3$ | 100 | 92 | 2.2 |
| Comparative example 1 | ----- | --- | 5% Pd/Al$_2$O$_3$ | 100 | 80 | 8.0 |
| Example 2 | Phenothiazine | 1000 | 5% Pd/C | 100 | 93 | 2.0 |
| Comparative example 2 | ----- | --- | 5% Pd/C | 100 | 82 | 8.6 |
| Example 3 | Phenyl-beta-naphthylamine | 500 | 5% Pd/Al$_2$O$_3$ | 100 | 90 | 2.7 |
| Example 4 | alpha-Naphthylamine | 100 | 5% Pd/Al$_2$O$_3$ | 100 | 92 | 3.0 |
| Example 5 | 2,6-Di-tert-butyl-p-cresol | 1000 | 5% Pt/C | 100 | 59 | 1.9 |
| Comparative example 3 | ----- | --- | 5% Pt/C | 100 | 48 | 7.9 |
| Example 6 | 2,4-Dimethyl-6-tert-butylphenol | 5000 | Raney Ni | 100 | 62 | 3.8 |
| Comparative example 4 | ----- | --- | Raney Ni | 100 | 53 | 6.8 |
| Example 7 | Hydroquinone | 500 | 5% Pd/Al$_2$O$_3$ | 100 | 84 | 4.3 |
| Example 8 | Thioalkylphenol | 500 | 5% Pd/Al$_2$O$_3$ | 100 | 80 | 6.7 |
| Example 9 | Triphenyl phosphite | 100 | 5% Pd/Al$_2$O$_3$ | 100 | 82 | 6.3 |
| Example 10 | Phenothiazine | 1000 | 5% Pd/C | 100 | 91 | 2.3 |
| Example 11 | alpha-Naphthylamine | 100 | 5% Pd/Al$_2$O$_3$ | 100 | 90 | 3.5 |

EP 0 293 127 B1

EP 0 293 127 B1

## Claims

1. A process for preparing glyoxylic acid comprising ;

a)     dissolving a maleic acid derivative selected from maleic acid, maleic anhydride, monoalkyl maleate and dialkyl maleate in a lower alkanol ;

b)     reacting said dissolved maleic acid derivative with an excess of ozone at a temperature not above 10°C to give an oxidized compound ;

c)     hydrogenating said oxidized compound in the presence of an antioxidant at a temperature not above 15°C over a transition metal catalyst or a platinum group metal catalyst to give glyoxylic acid hemiacetal;

d)     filtering the reaction mixture to remove the catalyst ;

e)     reacting the resultant hemiacetal with an excess of water after or without removing the solvent ; and

f)     evaporating the excess water or the excess water and solvent.

2. The process of claim 1, wherein the maleic acid derivative is maleic acid.

3. The process of claim 1 or claim 2, wherein the lower alkanol is methanol.

4. The process of any preceding claim, wherein the hydrogenation is conducted in the temperature range of 5°C to −40°C.

5. The process of any preceding claim, wherein the catalyst is a Raney type metal selected from nickel, iron, cobalt and copper.

6. The process of any of claims 1 to 4, wherein the catalyst is a powder of a metal selected from palladium, platinum, rhodium, ruthenium and iridium ; or one of these metals supported on alumina, silica, silica-alumina or activated carbon.

7. The process of any preceding claim, wherein the antioxidant is a quinone selected from hydroquinone, hydroquinone monomethyl ether and naphthoquinone.

8. The process of any of claims 1 to 6, wherein the antioxidant is an aromatic amine selected from the group consisting of phenothiazine, alpha-naphthylamine, N-phenyl-beta-naphthylamine, N,N′-diphenyl-p-phenylenediamine and N,N′-di-sec-butyl-p-phenylenediamine.

9. The process of any one of claims 1 to 6, wherein the antioxidant is a phenol selected from 2,6-di-tert-butyl-p-cresol, 2,6-di-tert-butylphenol, 2,4-dimethyl-6-tert-butylphenol, butylhydroxyanisole and 2,2′-methylenebis(4-methyl-6-tert-butylphenol).

10. The process of any of claims 1 to 6, wherein the antioxidant is a metal deactivator selected from thioalkylphenol or the calcium salt thereof, thioterpene, tributyl phosphite, triphenyl phosphite and calcium dithiophosphate.

## Patentansprüche

1. Verfahren zur Herstellung von Glyoxylsäure, **gekennzeichnet** durch

a)     Lösen eines aus Maleinsäure, Maleinanhydrid, Monoalkylmaleat und Dialkylmaleat in einem niedrigen oder unteren Alkanol gewählten Maleinsäurederivats,

b)     Reagieren des gelösten Maleinsäurederivats mit einem Überschuß Ozon bei einer Temperatur nicht über 10°C, um eine oxidierte Verbindung zu ergeben,

c)     Hydrogenieren der oxidierten Verbindung in Gegenwart eines Antioxidans bei einer Temperatur nicht über 15°C über einem Übergangsmetallkatalysator oder einem Metallkatalysator der Platingruppe, um Glyoxylsäurehemiazetal zu ergeben,

d)     Filtern des Reaktionsgemisches zwecks Entfernung des Katalysators,

e)     Reagieren des gewonnenen Hemiazetals mit einem Überschuß Wasser nach oder ohne Entfernen des Lösungsmittels und

f)     Verdampfen des überschüssigen Wassers oder des Wasserüberschusses und des Lösungsmittels.

2. Verfahren nach Anspruch 1, dadurch **gekennzeichnet**, daß das Maleinsäurederivat Maleinsäure ist.

3. Verfahren nach einem der vorhergehenden Ansprüche 1 oder 2, dadurch **gekennzeichnet**, daß das niedrige oder untere Alkanol Methanol ist.

4. Verfahren nach einem der vorhergehenden Ansprüche 1 bis 3, dadurch **gekennzeichnet**, daß die Hydro-

8

genierung im Temperaturbereich von 5°C bis –40°C durchgeführt wird.

5. Verfahren nach einem der vorhergehenden Ansprüche 1 bis 4, dadurch **gekennzeichnet**, daß als Katalysator ein aus der Gruppe Nickel, Eisen, Kobalt und Kupfer gewähltes Raney-Metall verwendet wird.

6. Verfahren fach einem der vorhergehenden Ansprüche 1 bis 4, dadurch **gekennzeichnet**, daß als Katalysator ein aus der Gruppe Palladium, Platin, Rhodium, Ruthen und Iridium gewähltes Metallpulver oder eines dieser von Aluminiumoxid, Siliziumoxid, Siliziumoxid/Aluminiumoxid'oder aktiviertem Kohlenstoff getragenen Metalle verwendet wird.

7. Verfahren nach einem.der vorhergehenden Ansprüche 1 bis 6, dadurch **gekennzeichnet**, daß als' Antioxidans ein aus der Gruppe Hydrochinon, Hydrochinonmonomethyläther und Naphthochinon gewähltes Chinon verwendet wird.

8. Verfahren nach einem der vorhergehenden Ansprüche 1 bis 6, dadurch **gekennzeichnet**, daß als Antioxidans ein aus der Gruppe Phenothiazin, alpha-Naphthylamin, N-Phenyl-beta-naphthylamin, N,N'-Diphenyl-p-phenylendiamin und N,N'-Di-sec-butyl-p-phenylendiamin gewähltes aromatisches Amin verwendet wird.

9. Verfahren nach einem der vorhergehenden Ansprüche 1 bis 6, dadurch **gekennzeichnet**, daß als Antioxidans ein aus der Gruppe 2,6-Di-tert-butyl-p-cresol, 2,6-Di-tert-butylphenol, 2,4-Dimethyl-6-tert-butylphenol, Butylhydrpxyanisol und 2,2'-Methylenbis-(4-methyl-6-tert-butylphenol) gewähltes Phenol verwendet wird.

10. Verfahren nach einem der vorhergehenden Ansprüche 1 bis, dadurch **gekennzeichnet**, daß als Antioxidans eine aus der Gruppe Thioalkylphenol oder dessen Kalziumsalz, Thioterpen, Tributylphosphit, Triphenylphosphit und Kalziumdithiophosphat gewählter Metall Deaktivator verwendet wird.

## Revendications

1. Procédé de préparation d'acide glycoxylique comprenant :

a)    la dissolution d'un dérivé de l'acide maléique choisi parmi l'acide maléique, l'anhydride maléique, un maléate de monoalkyle et un maléate de dialkyle en solution dans un alcanol inférieur.

b)    la mise en réaction de ce dérivé de l'acide maléique dissous avec un excès d'ozone à une température n'excédant pas 10°C, pour donner un composé ozonisé ;

c)    l'hydrogénation de ce composé ozonisé en présence d'un anti-oxydant à une température n'excédant pas 15°C sur un catalyseur à base d'un métal de transition ou un catalyseur constitué d'un métal du groupe du platine, pour donner un hémiacétal de l'acide glycoxylique ;

d)    filtration du mélange réactionnel pour éliminer le catalyseur ;

e)    mise en réaction de l'hémiacétal obtenu avec un excès d'eau après ou sans élimination du solvant ; et

f)    évaporation de l'excès d'eau ou de l'excès d'eau et de solvant.

2. Le procédé de la revendication 1, le dérivé d'acide maléique étant l'acide maléique.

3. Le procédé de la revendication 1 ou 2, l'alcanol inférieur étant le méthanol.

4. Le procédé de l'une quelconque des revendications précédentes, l'hydrogénation étant réalisée dans la fourchette de température allant de +5 à –40°C.

5. Le procédé de l'une quelconque des revendications précédentes, le catalyseur étant un métal du type Raney choisi parmi le nickel, le fer, le cobalt et le cuivre.

6. Le procédé de l'une quelconque des revendications 1 à 4, le catalyseur étant une poudre d'un métal choisi parmi le palladium, le platine, le rhodium, le ruthénium et l'iridium ; ou, un de ces métaux sur un support d'alumine, de silice, de silice-alumine ou de charbon actif.

7. Le procédé de l'une quelconque des revendications précédentes, l'anti-oxydant étant une quinone choisie parmi l'hydroquinone, l'éther de monométhylhydroquinone et la naphtoquinone.

8. Le procédé de l'une quelconque des revendications 1 à 6, l'anti-oxydant étant une amine aromatique choisie dans le groupe formé par la phénothiazine, l'α-naphthylamine, la N-phényl-β-naphthylamine, la N,N'-diphényl-p-phénylènediamine et la N,N'-di-sec-butyl-p-phénylènediamine.

9. Le procédé de l'une quelconque des revendications 1 à 6, l'anti-oxydant étant un phénol choisi parmi le 2,6-di- tert-butyl-p-crésol, le 2,4-dinéthyl-6-tertbutylphénol, le butylhydroxyanisole et le 2,2'-méthylène-bis(4-méthyl-6-tert-butylphénol).

10. Le procédé de l'une quelconque des revendications 1 à 6, l'anti-oxydant étant un désactivateur de métal ou un sel de calcium de celui-ci, un thioterpène, un phosphite de tributyle, un phosphite de triphényle et un dithiophosphate de calcium.